# EUROPEAN PATENT APPLICATION

(11) **EP 2 199 377 A1**
(43) Date of publication of application: **23.06.2010**
(21) Application number: 09175175.0
(22) Date of filing: 05.11.2009
(51) Int. Cl.: C10L 1/222, C10L 1/2387, C10L 1/22, C10L 1/14, C10L 10/14, C07C 213/04, C07C 213/00

(54) **Additives for fuel oils**

(30) Priority: 22.12.2008 EP 08022247
(71) Applicant: Infineum International Limited, Abingdon, Oxfordshire OX13 6BB (GB)
(72) Inventor: Tack, Robert, Abingdon, Oxfordshire OX13 6BB (GB)
(74) Representative: Capaldi, Michael Joseph

(57) **Abstract**

A fuel oil composition comprising a major amount of a fuel oil and a minor amount of a compound being the product of the reaction between (i) a primary or secondary hydrocarbyl-substituted amine or a mixture thereof and (ii) a species containing two or more epoxy groups being: a glycidyl ether (a) or (b), a glycidyl amine (c), a glycidyl ester (d) or a poly-epoxy alkane (e).

## Description

This invention relates to compounds effective to improve the low temperature properties of fuel oil compositions, and to improved fuel oil compositions, more especially to fuel oil compositions susceptible to wax formation at low temperatures. The invention also relates to fuel oil compositions susceptible to wax formation at low temperatures which further include detergent additives.

Fuel oils, whether derived from petroleum or from vegetable sources, contain components, e.g., n- alkanes or methyl n-alkanoates, that at low temperature tend to precipitate as large, platelike crystals or spherulites of wax in such a way as to form a gel structure which causes the fuel to lose its ability to flow. The lowest temperature at which the fuel will still flow is known as the pour point.

As the temperature of a fuel falls and approaches the pour point, difficulties arise in transporting the fuel through lines and pumps. Further, the wax crystals tend to plug fuel lines, screens, and filters at temperatures above the pour point. These problems are well recognised in the art, and various additives have been proposed, many of which are in commercial use, for depressing the pour point of fuel oils. Similarly, other additives have been proposed and are in commercial use for reducing the size and changing the shape of the wax crystals that do form. Smaller size crystals are desirable since they are less likely to clog a filter. The wax from a diesel fuel, which is primarily an alkane wax, crystallizes as platelets. Certain additives inhibit this and cause the wax to adopt an acicular habit, the resulting needles being more likely to pass through a filter, or form a porous layer of crystals on the filter, than are platelets. Other additives may also have the effect of retaining the wax crystals in suspension in the fuel, reducing settling and thus also assisting in prevention of blockages. These types of additives are often termed 'wax anti-settling additives' (WASA).

Many additives have been described over the years for enhancing engine cleanliness, e.g. for reducing or removing deposits in the intake system (e.g. carburetors, intake manifold, inlet valves) or combustion chamber surfaces of spark-ignition engines, or for reducing or preventing injector nozzle fouling in compression-ignition engines.

For example, UK Patent specification No. 960,493 describes the incorporation of metal-free detergents, in the form of polyolefin-substituted succinimides of tetraethylene pentamine, in base fuels for internal combustion engines. The use of such metal-free detergents is now widespread. Most commonly used are polyisobutylene substituted succinimides which are the reaction products of polyisobutylene substituted acylating agents such as succinic acid or anhydride with polyamines. Such materials and their methods of production will be known to those skilled in the art.

The trend in modern diesel engine technology is to increase power output and efficiency by increasing injection pressures and decreasing injector nozzle diameters. Under these conditions, the build up of injector deposits is more likely and the deposition which occurs is more severe. This has led fuel manufacturers to produce new types of fuels which are often sold as 'premium' grades and promoted as being especially effective to improve engine cleanliness. To meet this performance claim, such premium fuels usually contain significantly higher levels of detergent than non-premium grade fuels.

Whilst largely effective with regard to engine cleanliness, a drawback has been identified with the use of high levels of detergent in fuel oils. Specifically, it has been observed that the presence of high levels of polyamine detergent species in premium grade fuels can interfere with the cold-flow performance of wax anti-settling additives when these are also present in the fuel. So, although the fuel may be satisfactory from an engine cleanliness viewpoint, its low temperature properties, in terms of wax anti-settling and cold filter plugging point (CFPP) may not be adequate.

US 4,322,220 discloses an additive for hydrocarbon fuels and lubricating oils which is the reaction product of a glycidyl ether compound of the formula: 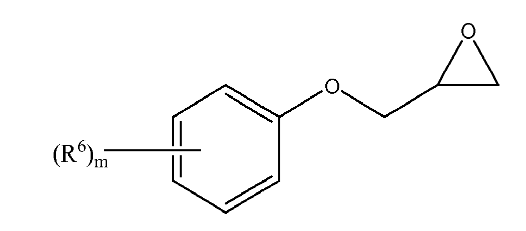 where R⁶ is an aliphatic hydrocarbon group containing at least 8 carbon atoms and m is 1-3 and a primary or secondary monoamine or polyamine. The compounds are described to provide carburetor induction system and combustion chamber detergency and rust inhibition.

The present invention is based on the discovery of a group of compounds which are effective as wax anti-settling additives in fuel oils. As well as providing fuel oils with improved low temperature properties, the compounds of the invention have the added advantage that their potency is much less affected by the presence of conventional fuel oil detergents.

Thus in accordance with a first aspect, the present invention provides a fuel oil composition comprising a major amount of a fuel oil and a minor amount of a compound being the product of the reaction between (i) a primary or secondary hydrocarbyl-substituted amine or a mixture thereof and (ii) a species containing two or more epoxy groups being:
(a) a glycidyl ether of general formula I: 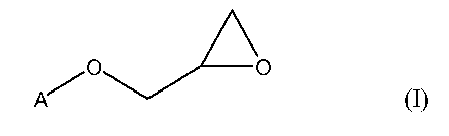 where A represents a moiety of formula II: 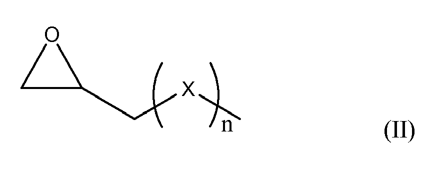 where X represents R or OR; where R represents a linear or branched alkyl linkage, preferably of between 1 and 12 carbon atoms or a polyalkylene glycol segment of number average molecular weight of between 200 and 1000; and n is from 0 to 20; or
(b) a glycidyl ether of general formula III:

   CB₂DD' (III)

   where B is a moiety of formula II where X is OR and n is at least 1, and where D and D' are independently either the same as B, a substituted or unsubstituted linear or branched alkyl group or hydrogen; or
(c) a glycidyl amine of general formula IV:

   ANR³R⁴ (IV)

   where A is a moiety of formula (II) as above and where R³ and R⁴ may be the same or different and are independently chosen from hydrogen, a substituted or unsubstituted linear or branched alkyl group, a substituted or unsubstituted cyclic aliphatic or aromatic group, or a moiety of formula (II), provided that at least one of R³ or R⁴ is a moiety of formula (II); or
(d) a glycidyl ester of general formula (V) 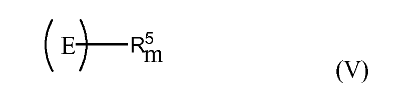 where E is a substituted or unsubstituted linear or branched alkyl group, a substituted or unsubstituted cyclic aliphatic or aromatic group, nitrogen or a tertiary amine or tertiary polyamine group, R⁵ is a moiety of formula (VI): 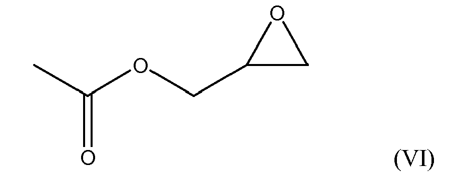 and m is an integer from 2 to 10, preferably from 2 to 5; or
(e) a poly-epoxy alkane.

Preferably, at least 10mol% of reactant (i) comprises a secondary hydrocarbyl-substituted amine, more preferably at least 20mol%, for example at least 30mol%.

In a particularly preferred embodiment, the compound of the invention is the product of the reaction between (i) a secondary hydrocarbyl-substituted amine or a mixture thereof with a primary hydrocarbyl-substituted amine, wherein at least 10mol% of the mixture comprises a secondary hydrocarbyl-substituted amine, and a species (ii) containing two epoxy groups.

In the case where species (ii) contains three or more epoxy groups, it is preferred that the amount of primary amine in reactant (i) is minimised. More preferably in the case where species (ii) has three or more epoxy groups, reactant (i) comprises a majority, that is greater than 50%, preferably greater than 75% of secondary hydrocarbyl-substituted amine or consists essentially of secondary hydrocarbyl-substituted amine.

Conveniently, the reaction mixture provides one mole of amine for each mole of epoxy groups although those skilled in the art will appreciate that these amounts may be varied as desired. Reaction is facile and may be effected by simply heating the mixture of reactants for a given time. Temperatures of between 80 and 160°C, e.g. 140°C, have been found to be suitable.
Reaction times of a few hours are generally adequate. Usually no solvent is required however, if desired the reaction may of course be carried out in a solvent. Toluene or xylene are suitable solvents.

In accordance with a second aspect, the present invention provides the use of a compound according to the first aspect to improve the low temperature properties of a fuel oil composition.

The various features of the invention, which are applicable as appropriate to all aspects will now be described in more detail.

### (i) The hydrocarbyl-substituted amine

Preferably the amine is of the formula NHR¹R², where R¹ independently represents a hydrocarbyl group, such as an alkyl group, containing from 8 to 40 carbon atoms, and R² independently represents hydrogen or a hydrocarbyl group, such as an alkyl group, containing up to 40 carbon atoms.

Examples of primary amines include decylamine, dodecylamine, tetradecyl amine, hexadecylamine and octadecylamine. Examples of secondary amines include dioctadecyl amine and methyl-behenyl amine. Amine mixtures are suitable such as those derived from natural materials. A preferred amine is a secondary hydrogenated tallow amine where R¹and R² are alkyl groups derived from hydrogenated tallow fat composed of approximately 4% C₁₄, 31% C₁₆ and 59% C₁₈. Also preferred is cocoamine.

### (ii) The species containing one or more epoxy groups

Non-limiting examples of glycidyl ethers (a) and (b) include: 1,4-butanediol diglycidyl ether, ethylene glycol diglycidyl ether, polyalkyleneglycol diglycidyl ethers such as polyethyleneglycol diglycidyl ether and polypropyleneglycol diglycidyl ether where the polyalkyleneglycol segment has a number average molecular weight of between 200 and 1,000, linear and branched alkyl glycidyl ethers such as glycerine triglycidyl ether, glycerol propoxy triglycidyl ether and trimethylol propane triglycidyl ether.

Non limiting examples of glycidyl amines (c) include: N,N-diglycidyl aniline and N,N-diglycidyl-4-glycidyloxyaniline.

Non limiting examples of glycidyl esters (d) include: di-(2,3-epoxy-propyl) cyclohexane-1,4-dicarboxylate, di-(2,3-epoxy-propyl) adipate, di-(2,3-epoxy-propyl) isophthalate, di-(2,3-epoxy-propyl) succinate, di-(2,3-epoxy-propyl) azeleate, di-(2,3-epoxy-propyl) terephthalate, tri-(2,3-epoxy-propyl)benzene tricarboxylate, tri-(2,3-epoxy-propyl)benzene citrate, tri-(2,3-epoxypropyl)benzene nitrilo-triacetate, tetra-(2,3-epoxy-propyl)benzene tetracarboxylate and tetra-(2,3-epoxy-propyl)benzene ethylene-diamine-tetracarboxylate.

Suitable poly-epoxy alkanes (e) are di-epoxy alkanes and tri-epoxy alkanes, for example 1,2,7,8 diepoxy octane.

A discussed above, the compounds of the present invention have the additional advantage that they do not lose potency when used in combination with conventional fuel oil detergents. Thus in preferred embodiments, the fuel oil composition further comprises a fuel oil detergent.

In accordance with a third aspect, the present invention provides the use of a combination of a fuel oil detergent and a compound effective as a wax anti-settling additive to improve the detergency properties and low temperature properties of a fuel oil composition, wherein the low temperature properties of the fuel oil composition are at least substantially similar to the low temperature characteristics of the fuel oil composition comprising the compound effective as a wax anti-settling additive in the absence of the fuel oil detergent, the use comprising employing as the compound effective as a wax anti-settling additive, a compound according to the first aspect.

The term 'at least substantially similar' in the context of this third aspect should be understood to mean that the presence of the detergent does not have a significant negative influence on at least one of the low temperature characteristics (e.g. CFPP or wax anti-settling) of the fuel oil containing the compound of the invention compared to the situation where the detergent is absent. It is not required that the low temperature characteristics are improved in absolute terms, merely that they are not adversely affected on a practical level. Of course, an improvement in absolute terms is also within the scope of the present invention.

Preferably, the fuel oil detergent comprises a polyamine detergent. A preferred class of polyamine detergents are those made by reacting an acylating agent having a hydrocarbyl substituent of at least 10 carbon atoms and a nitrogen compound characterized by the presence of at least one -NH- group. Typically, the acylating agent will be a mono- or polycarboxylic acid (or reactive equivalent thereof) such as a substituted succinic or propionic acid and the amino compound will be a polyamine or mixture of polyamines, most typically, a mixture of ethylene polyamines. The amine also may be a hydroxyalkyl-substituted polyamine. The hydrocarbyl substituent in such acylating agents preferably averages at least about 30 or 50 and up to about 200 carbon atoms.

Illustrative of hydrocarbyl substituent groups containing at least 10 carbon atoms are n-decyl, n-dodecyl, tetrapropenyl, n-octadecyl, oleyl, chlorooctadecyl, triicontanyl, etc. Generally, the hydrocarbyl substituents are made from homo- or interpolymers (e.g. copolymers, terpolymers) of mono- and di-olefins having 2 to 10 carbon atoms, such as ethylene, propylene, 1-butene, isobutene, butadiene, isoprene, 1-hexene, 1-octene, etc. Typically, these olefins are 1-monoolefins. This substituent can also be derived from the halogenated (e.g. chlorinated or brominated) analogs of such homo-or interpolymers.

The hydrocarbyl substituents are predominantly saturated. The hydrocarbyl substituents are also predominantly aliphatic in nature, that is, they contain no more than one non-aliphatic moiety (cycloalkyl, cycloalkenyl or aromatic) group of 6 or less carbon atoms for every 10 carbon atoms in the substituent. Usually, however, the substituents contain no more than one such non-aliphatic group for every 50 carbon atoms, and in many cases, they contain no such non-aliphatic groups at all; that is, the typically substituents are purely aliphatic. Typically, these purely aliphatic substituents are alkyl or alkenyl groups.

A preferred source of the substituents are poly(isobutene)s obtained by polymerization of a C₄ refinery stream having a butene content of 35 to 75 weight per cent and isobutene content of 30 to 60 weight per cent in the presence of a Lewis acid catalyst such as aluminum trichloride or boron trifluoride. These polybutenes predominantly contain monomer repeating units of the configuration -C(CH₃)₂CH₂-.

The hydrocarbyl substituent is attached to the succinic acid moiety or derivative thereof via conventional means, for example the reaction between maleic anhydride and an unsaturated substituent precursor such as a polyalkene, as described for example in EP-B-0 451 380.

One procedure for preparing the substituted succinic acylating agents involves first chlorinating the polyalkene until there is an average of at least about one chloro group for each molecule of polyalkene. Chlorination involves merely contacting the polyalkene with chlorine gas until the desired amount of chlorine is incorporated into the chlorinated polyalkene. Chlorination is generally carried out at a temperature of about 75°C to about 125°C. If desired, a diluent can be used in the chlorination procedure. Suitable diluents for this purpose include poly- and perchlorinated and/or fluorinated alkanes and benzenes.

The second step in the procedure is to react the chlorinated polyalkene with the maleic reactant at a temperature usually within the range of about 100°C to about 200°C. The mole ratio of chlorinated polyalkene to maleic reactant is usually about 1:1. However, a stoichiometric excess of maleic reactant can be used, for example, a mole ratio of 1:2. If an average of more than about one chloro group per molecule of polyalkene is introduced during the chlorination step, then more than one mole of maleic reactant can react per molecule of chlorinated polyalkene. It is normally desirable to provide an excess of maleic reactant; for example, an excess of about 5% to about 50%, for example 25% by weight. Unreacted excess maleic reactant may be stripped from the reaction product, usually under vacuum.

Another procedure for preparing substituted succinic acid acylating agents utilizes a process described in U.S. Pat. No. 3,912,764 and U.K. Pat. No. 1,440,219. According to that process, the polyalkene and the maleic reactant are first reacted by heating them together in a direct alkylation procedure. When the direct alkylation step is completed, chlorine is introduced into the reaction mixture to promote reaction of the remaining unreacted maleic reactants. According to the patents, 0.3 to 2 or more moles of maleic anhydride are used in the reaction for each mole of polyalkene. The direct alkylation step is conducted at temperatures to 180°C to 250°C. During the chlorine-introducing stage, a temperature of 160°C to 225°C is employed.

The attachment of the hydrocarbyl substituent to the succinic moiety may alternatively be achieved via the thermally-driven 'ene' reaction, in the absence of chlorine. Use of such a material as the acylating agent leads to products having particular advantages; for example, chlorine-free products having excellent detergency and lubricity properties. In such products, the reactant is preferably formed from a polyalkene having at least 30% preferably 50% or more such as 75% of residual unsaturation in the form of terminal, e.g. vinylidene, double bonds.

Suitable polyamines are those comprising amino nitrogens linked by alkylene bridges, which amino nitrogens may be primary, secondary and/or tertiary in nature. The polyamines may be straight chain, wherein all the amino groups will be primary or secondary groups, or may contain cyclic or branched regions or both, in which case tertiary amino groups may also be present. The alkylene groups are preferably ethylene or propylene groups, with ethylene being preferred. Such materials may be prepared from the polymerization of lower alkylene diamines such as ethylene diamine, a mixture of polyamines being obtained, or via the reaction of dichloroethane and ammonia.

Specific examples of the polyalkylene polyamines (1) are ethylene diamine, tetra(ethylene)pentamine, tri-(trimethylene)tetramine, and 1,2-propylene diamine. Specific examples of hydroxyalkyl-substituted polyamines include N-(2-hydroxyethyl) ethylene diamine, N,N¹-bis-(2-hydroxyethyl) ethylene diamine, N-(3-hydroxybutyl) tetramethylene diamine, etc. Specific examples of the heterocyclic-substituted polyamines (2) are N-2-aminoethyl piperazine, N-2 and N-3 amino propyl morpholine, N-3-(dimethylamino) propyl piperazine, 2-heptyl-3-(2-aminopropyl) imidazoline, 1,4-bis (2-aminoethyl) piperazine, 1-(2-hydroxy ethyl) piperazine, and 2-heptadecyl-1-(2-hydroxyethyl)-imidazoline, etc. Specific examples of the aromatic polyamines (3) are the various isomeric phenylene diamines, the various isomeric naphthalene diamines, etc.

Many patents have described suitable polyamine detergents including US Patents 3 172 892; 3 219 666; 3 272 746; 3 310 492; 3 341 542; 3 444 170; 3 455 831; 3 455 832; 3 576 743; 3 630 904; 3 632 511; 3 804 763 and 4 234 435, and including European patent applications EP 0 336 664 and EP 0 263 703. A typical and preferred compound of this class is that made by reacting a poly(isobutylene)-substituted succinic anhydride acylating agent (e.g. anhydride, acid, ester, etc.) wherein the poly(isobutene) substituent has between about 50 to about 200 carbon atoms with a mixture of ethylene polyamines having 3 to about 10 amino nitrogen atoms per ethylene polyamine and about 1 to about 6 ethylene groups.

The polyamine component may be defined by the average number of nitrogen atoms per molecule of the component, which may preferably be in the range of 4 to 8.5, more preferably 6.8 to 8, especially 6.8 to 7.5 nitrogens per molecule.

Also suitable are materials made from amine mixtures comprising polyamines having seven and eight, and optionally nine, nitrogen atoms per molecule (so-called 'heavy' polyamines).

Preferably, the polyamine mixture comprises at least 45% and preferably 50% by weight of polyamines having seven nitrogen atoms per molecule, based on the total weight of polyamines. In addition to polyamine mixtures, single species may also be used, for example TEPA and TETA.

A preferred polyamine detergent comprises the reaction product between a poly(isobutene) substituted succinic anhydride acylating agent with a polyamine or mixture of polyamines as hereinbefore described. Preferably, the poly(isobutene) has a number average molecular weight (Mn) of about 400-2500, preferably 400-1300, such as about 950.

### The fuel oil

The fuel oil may be, e.g., a petroleum-based fuel oil, especially a middle distillate fuel oil. Such distillate fuel oils generally boil within the range of from 110°C to 500°C, e.g. 150°C to 400°C.

The invention is applicable to middle distillate fuel oils of all types, including the broad-boiling distillates, i.e., those having a 90%-20% boiling temperature difference, as measured in accordance with ASTM D-86, of 50°C or more.

The fuel oil may comprise atmospheric distillate or vacuum distillate, cracked gas oil, or a blend in any proportion of straight run and thermally and/or catalytically cracked distillates. The most common petroleum distillate fuels are kerosene, jet fuels, diesel fuels, heating oils and heavy fuel oils. The heating oil may be a straight atmospheric distillate, or may also contain vacuum gas oil or cracked gas oil or both. The fuels may also contain major or minor amounts of components derived from the Fischer-Tropsch process. Fischer-Tropsch fuels, also known as FT fuels, include those that are described as gas-to-liquid fuels, coal and/or biomass conversion fuels. To make such fuels, syngas (CO + H₂) is first generated and then converted to normal paraffins and olefins by a Fischer-Tropsch process. The normal paraffins may then be modified by processes such as catalytic cracking/reforming or isomerisation, hydrocracking and hydroisomerisation to yield a variety of hydrocarbons such as iso-paraffins, cyclo-paraffins and aromatic compounds. The resulting FT fuel can be used as such or in combination with other fuel components and fuel types such as those mentioned in this specification. The above mentioned low temperature flow problem is most usually encountered with diesel fuels and with heating oils. The invention is also applicable to fuel oils containing fatty acid methyl esters derived from vegetable oils, for example, rapeseed methyl ester, soya-oil methyl ester, palm-oil methyl ester and the like, either used alone or in admixture with a petroleum distillate oil.

In an embodiment of all aspects of the present invention, the fuel oil comprises at least 2%, preferably at least 5% by weight of fatty acid methyl esters derived from vegetable oils.

The fuel oil is preferably a low sulphur content fuel oil. Typically, the sulphur content of the fuel oil will be less than 500ppm (parts per million by weight). Preferably, the sulphur content of the fuel will be less than 100ppm, for example, less than 50ppm. Fuel oils with even lower sulphur contents, for example less that 20ppm or less than 10ppm are also suitable.

### Treat Rates

The amounts of each component present in the fuel oil will depend on the nature of the species used, the properties of the fuel oil and the low temperature performance required.

The amount of compound used according to the invention will typically be in the range of 10 - 500 ppm, preferably 10 - 200 ppm by weight based on the weight of the fuel oil.

When present, typically, the amount of fuel oil detergent in the fuel oil composition will be in excess of 50ppm by weight based on the weight of the fuel oil, for example in excess of 75ppm by weight or 100ppm by weight. Some premium diesel fuels may contain up to 500 ppm by weight of polyamine detergent. This can be compared to a treat rate of around 10 -75 ppm for non-premium diesel fuels.

### Other additives

It is commonplace in the art to use compounds effective as a wax anti-settling additives in combination with other additional cold-flow improving additives. Suitable materials will be well known to those skilled in the art and include for example, ethylene-unsaturated ester copolymers such as ethylene-vinyl acetate copolymers, comb polymers such as fumarate-vinyl acetate copolymers, hydrocarbon polymers such as hydrogenated polybutadiene polymers, ethylene/1-alkene copolymers, and similar polymers.

Also suitable are condensate species such as alkyl-phenol formaldehyde condensates as described in EP 0 857 776 B1, or hydroxy-benzoate formaldehyde condensates as described in EP-A-1 482 024.

The present invention contemplates the addition of such additional cold-flow improving additives; their application in terms of treat rate being also well known to those skilled in the art. In an embodiment of all aspects of the invention, the fuel oil further comprises one or more of an ethylene-unsaturated ester copolymer, a fumarate-vinyl acetate copolymer, an alkyl-phenol formaldehyde condensate, a hydroxy-benzoate formaldehyde condensate and a hydrocarbon polymer.

### Evaluation of low temperature properties.

As is known in the art, there are a number of methods which can be used to determine the low temperature properties of a fuel oil. Preferably, the low temperature properties are as determined by measuring ΔCP, CFPP, or both. Preferably, the low temperature properties improved in the present invention are ΔCP, CFPP or both.

ΔCP is a measurement of the propensity of the wax content of a fuel oil to settle and thus a determination of the effectiveness of a wax anti-settling additive. To determine ΔCP, the cloud point (CP) of a base fuel oil is measured. The wax anti-settling additive under study is then added to the base fuel and the sample cooled to a temperature below the measured CP. This temperature may vary, in Germany a temperature of -13°C is commonly used, in South Korea it may be -15 or -20°C and a value of -18°C is also often used. After leaving the fuel oil sample for a time to allow any wax to settle, the CP of the bottom 20% by volume of the sample is measured. The difference between this measurement and the value obtained for the base fuel is ΔCP. A small value, preferably around zero, of ΔCP indicates good wax dispersancy.

CFPP is a standard industry test to evaluate the ability of a fuel oil sample to flow through a filter at reduced temperature. The test which is carried out by the procedure described in detail in "Jn. Of the Institute of Petroleum ", vol. 52, No. 510 (1996), pp 173-285, is designed to correlate with the cold flow of a middle distillate in automotive diesels. In brief, a sample of the oil to be tested (40 cm³) is cooled in a bath which is maintained at about -34°C to give linear cooling at about 1°C/min. Periodically (at each one degree centigrade starting from above the cloud point), the oil is tested for its ability to flow through a fine screen in a prescribed time period using a test device which is a pipette to whose lower end is attached an inverted funnel which is positioned below the surface of the oil to be tested. Stretched across the mouth of the funnel is a 350 mesh screen having an area defined by a 12 mm diameter. The periodic tests are initiated by applying a vacuum to the upper end of the pipette whereby oil is drawn through the screen up into the pipette to a mark indicating 20 cm³ of oil. After each successful passage, the oil is returned immediately to the CFPP tube. The test is repeated with each one degree drop in temperature until the oil fails to fill the pipette within 60 seconds, the temperature at which failure occurs being reported as the CFPP temperature.

The invention will now be described by way of example only.

Compounds according to the invention were made as detailed in Table 1 below. The compounds were prepared by reacting 1 mole of the amine for each mole of epoxy group present. The reactants were mixed and heated to 140°C for 4 hours.

**Table 1**

| No. | (i) Amine species | (ii) epoxy species |
|---|---|---|
| 1 | di-hydrogenated tallow amine | glycerol propoxy triglycidyl ether |
| 2 | di-hydrogenated tallow amine | tri-methylol propane triglycidyl ether |
| 3 | di-hydrogenated tallow amine | polyethylene glycol diglycidyl ether |
| 4 | di-hydrogenated tallow amine | cyclohexane dicarboxy glycidyl ester |
| 5 | di-hydrogenated tallow amine | 1,4-butanediol diglycidyl ether |
| 6 | di-hydrogenated tallow amine | polypropylene glycol diglycidyl ether |
| 7 | di-hydrogenated tallow amine | N,N-diglycidyl-4-glycidyloxyaniline |
| 8 | di-hydrogenated tallow amine/ di-cocoamine (50:50) | cyclohexane dicarboxy glycidyl ester |
| 9 | di-hydrogenated tallow amine/ octadecylamine (equimolar) | 1,4-butanediol diglycidyl ether |
| 10 | di-hydrogenated tallow amine/ | 1,4-butanediol diglycidyl ether |
| | di-cocoamine/octadecylamine (equimolar) | |

Table 2 below details the results of testing of compounds according to the present invention for both CFPP and ΔCP. The fuel used was low sulphur-content diesel fuel containing 5% by weight of soya-oil methyl ester, with a CFPP of -11.0°C. The diesel fuel portion had an initial boiling point of 195°C and a final boiling point of 350°C. Each compound according to the invention was used in an amount of 100 ppm by weight based on the weight of the fuel. Also added in each case was 300ppm of a conventional middle distillate flow improver package being the combination of an ethylene vinyl acetate copolymer, an ethylene vinyl acetate/vinyl 2-ethylhexanoate terpolymer and a hydrogenated diblock copolymer of butadiene.

**Table 2**

| **Compound** | **CFPP/°C** | Δ**CP** |
|---|---|---|
| none | -18.5 | 8.9 |
| 1 | -27.0 | 0.1 |
| 2 | -23.5 | 0.6 |
| 3 | -23.0 | 0.0 |
| 4 | -20.25 | 1.9 |
| 5 | -20.0 | 7.7 |
| 6 | -19.0 | 5.4 |
| 7 | -18.5 | 3.0 |

Table 3 below, in the second and third columns, shows results for the compounds of the invention when formulated into an additive package. Two parts by weight of the compounds of the invention were combined with one part by weight each of a fumarate/vinyl acetate copolymer and an alkylphenol formaldehyde condensate. This mixture was used in the same fuel as described above in an amount of 150ppm by weight. Also present for each test was 400ppm by weight of the conventional middle distillate flow improver package described above.

Also shown in Table 3, in the final two columns, is the effect of the addition to the fuel of 100ppm by weight of a conventional diesel fuel detergent being a polyisobutylene-substituted succinimide where the polyisobutylene substituent was of ca. 950 molecular weight and the amine was a mixture of polyethylene polyamines having an average of 6-8 nitrogen atoms per molecule. The table also provides results for a "Reference WASA". This was a product not according to the present invention being formed by the reaction of phthalic anhydride with di-hydrogenated tallow amine. It was tested in the same manner as the compounds of the invention.

**Table 3**

| **Compound** | **CFPP/°C** | Δ**CP** | **CFPP/°C** | Δ**CP** |
|---|---|---|---|---|
| 9 | -26.0 | 1.5 | -25.0 | 0.6 |
| 2 | -26.5 | 0.0 | -23.5 | -0.4 |
| 4 | -28.0 | 7.4 | -23.0 | 8.0 |
| 8 | -22.7 | 8.0 | -23.0 | 8.5 |
| 10 | -24.0 | 1.3 | -22.75 | -0.2 |
| 1 | -24.0 | 1.4 | -22.5 | 7.1 |
| 7 | -21.5 | 1.7 | -22.0 | 0.6 |
| 6 | -23.7 | 7.7 | -20.5 | 8.2 |
| 3 | -23.5 | 1.2 | -19.7 | 0.0 |
| Reference WASA | -25.25 | 0.3 | -19.5 | 8.9 |

The data obtained show that the compounds of the invention are effective to improve the low temperature properties of the fuel. Both good CFPP and ΔCP values were obtained and in all cases, one or both of these values was improved compared to the fuel treated with the conventional middle distillate flow improver alone (Table 2).

In the majority of cases, the addition of the conventional detergent (Table 3) did not have a detrimental effect on the potency of the compounds. The Reference WASA performed poorly, losing a significant amount of both CFPP and ΔCP performance when the detergent was added.

## Claims

1. A fuel oil composition comprising a major amount of a fuel oil and a minor amount of a compound being the product of the reaction between (i) a primary or secondary hydrocarbyl-substituted amine or a mixture thereof and (ii) a species containing two or more epoxy groups being:
(a) a glycidyl ether of general formula I: 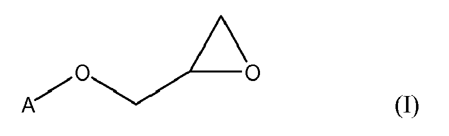 where A represents a moiety of formula II: 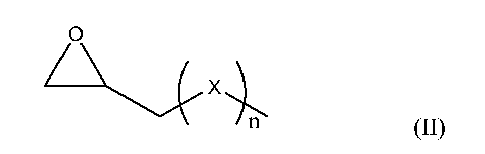 where X represents R or OR; where R represents a linear or branched alkyl linkage, preferably of between 1 and 12 carbon atoms or a polyalkylene glycol segment of number average molecular weight of between 200 and 1000; and n is from 0 to 20; or
(b) a glycidyl ether of general formula III:
CB₂DD' (III)
where B is a moiety of formula II where X is OR and n is at least 1, and where D and D' are independently either the same as B, a substituted or unsubstituted linear or branched alkyl group or hydrogen; or
(c) a glycidyl amine of general formula IV:
ANR³R⁴ (IV)
where A is a moiety of formula (II) as above and where R³ and R⁴ may be the same or different and are independently chosen from hydrogen, a substituted or unsubstituted linear or branched alkyl group, a substituted or unsubstituted cyclic aliphatic or aromatic group, or a moiety of formula (II), provided that at least one of R³ or R⁴ is a moiety of formula (II); or
(d) a glycidyl ester of general formula (V) 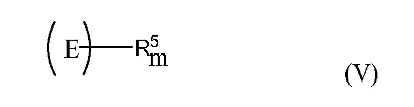 where E is a substituted or unsubstituted linear or branched alkyl group, a substituted or unsubstituted cyclic aliphatic or aromatic group, nitrogen or a tertiary amine or tertiary polyamine group, R⁵ is a moiety of formula (VI): 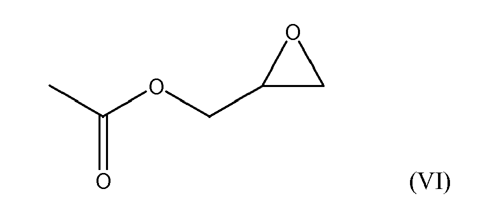 and m is an integer from 2 to 10, preferably from 2 to 5; or
(e) a poly-epoxy alkane.

2. A fuel oil composition according to claim 1 wherein species (ii) is a glycidyl ether (a) or a glycidyl ether (b).

3. A fuel oil composition according to claim 1 or claim 2 wherein (i) is of the formula NHR¹R², where R¹ independently represents a hydrocarbyl group, such as an alkyl group, containing from 8 to 40 carbon atoms, and R² independently represents hydrogen or a hydrocarbyl group, such as an alkyl group, containing up to 40 carbon atoms.

4. A fuel oil composition according to any preceding claim wherein at least 10mol% of (i) comprises a secondary hydrocarbyl-substituted amine.

5. A fuel oil composition according to any preceding claim further comprising one or more of an ethylene-unsaturated ester copolymer, a fumarate-vinyl acetate copolymer, an alkyl-phenol formaldehyde condensate, a hydroxy-benzoate formaldehyde condensate and a hydrocarbon polymer.

6. A fuel oil composition according to any preceding claim further comprising a fuel oil detergent.

7. A fuel oil composition according to claim 6 where in the fuel oil detergent comprises a polyamine detergent.

8. The use of a compound as defined in any of claims 1 to 4 to improve the low temperature properties of a fuel oil composition.

9. The use of the combination of a fuel oil detergent and an oil-soluble compound effective as a wax anti-settling additive to improve the detergency properties and low-temperature properties of a fuel oil composition, wherein the low temperature properties of the fuel oil composition are substantially similar to the low temperature properties of the fuel oil composition comprising the oil-soluble compound effective as a wax anti-settling additive in the absence of the fuel oil detergent, the use comprising employing as the oil-soluble wax anti-settling additive a compound according to any of claims 1 to 4.

10. The use according to claim 9 wherein the amount of fuel oil detergent in the fuel oil composition is in excess of 50ppm by weight and the amount of oil-soluble compound effective as a wax anti-settling additive in the fuel oil composition is in the range of 10-500ppm by weight, both amounts based on the weight of the fuel oil.
